# EUROPEAN PATENT SPECIFICATION

(11) **EP 4 034 178 B1**
(45) Date of publication and mention of the grant of the patent: **15.01.2025**
(21) Application number: 20792782.3
(22) Date of filing: 22.09.2020
(51) Int. Cl.: A61L 2/10, A61L 2/18, A61M 25/00

(54) **REUSABLE URINARY CATHETER PRODUCTS**
WIEDERVERWENDBARE HARNKATHETERPRODUKTE
PRODUITS DE CATHÉTER URINAIRE RÉUTILISABLES

(30) Priority: 24.09.2019 US 201962905044 P
(43) Date of publication of application: 03.08.2022
(60) Divisional application: 24197151.4
(73) Proprietor: Hollister Incorporated, Libertyville, IL 60048-3781 (US)
(72) Inventor: MURRAY, Michael G., Libertyville, IL 60048 (US); DODD, Ian, Libertyville, IL 60048 (US); MONTES de OCA BALDERAS, Horacio, Libertyville, IL 60048 (US); CULLUM, Malford E., Libertyville, IL 60048 (US); KUMAR, Varun, Libertyville, IL 60048 (US)
(74) Representative: FRKelly
(86) International application number: PCT/US2020/052027
(87) International publication number: WO 2021/061661

(56) References cited:
- WO-A1-2014/159855
- WO-A1-2020/223043
- US-A- 5 310 524
- US-A1- 2015 231 287
- US-A1- 2019 328 915
- US-B2- 9 550 005

## Description

The present application claims the benefit of and priority to U.S. Provisional Application No. 62/905,044, filed September 24, 2019.

### Field of the Disclosure

The present disclosure generally relates to urinary catheters. More particularly, the present disclosure relates to reusable urinary catheter products.

### Background

Catheters are used to treat many different types of medical conditions and typically include an elongated shaft that is inserted into and through a passageway or lumen of the body. Catheters, and in particular intermittent catheters, are commonly used by those who suffer from various abnormalities of the urinary system, such as urinary incontinence. With the advent of intermittent catheters, individuals with urinary system abnormalities can self-insert and self-remove intermittent catheters several times a day.

Urinary catheters are frequently provided as disposable, single-use items. A user will remove the catheter from a package, use the catheter once, and then dispose of the catheter and the package. Reusable urinary catheters could, thus, be advantageous in reducing the amount of waste created by the use of disposable catheters, but there are various challenges associated with the use of reusable catheters (including storage, transport, and sterilization) that must be overcome before widespread acceptance and use of reusable catheters. US 2019/328915A1 and WO 2020/223043 A1 disclose an at-home sterilization, data acquisition, and deposition device for sterilizing multiple medical devices.

There is a need for reusable catheter products and methods of sterilizing the same.

### Summary

According to the presently claimed invention, a reusable urinary catheter product includes a case comprising a body and a re-closable cap. The body defines a first compartment and a second compartment wherein the first compartment and second compartment are separated by a barrier that allows the passage of sterilizing light. A urinary catheter is located in the first compartment. A sterilizing light source is located in the second compartment, wherein sterilizing light emitted from the light source passes through the barrier and into the second compartment.

### Brief Description of the Drawings

Fig. 1 is perspective view of a reusable catheter product;
Fig. 2 is a partial perspective view of the reusable catheter product of Fig. 1;
Fig. 3 is a cross-sectional view of the reusable catheter product of Fig. 1;
Fig. 4 is a cross-sectional view of an alternative embodiment of the reusable catheter product of Fig. 1;
Fig. 5 is a side elevational view of another embodiment of a reusable catheter product;
Fig. 6 is a bottom perspective view of the top portion of the reusable catheter product of Fig. 5;
Fig. 7 is a top perspective view of the bottom portion of the reusable catheter product of Fig. 5;
Fig. 8 is a perspective view of another embodiment of a reusable catheter product;
Fig. 9 is a perspective view of one embodiment of a flushing chamber of the reusable catheter product shown in Fig. 8;
Fig. 10 is a perspective view of one embodiment of the cradle of the reusable catheter product shown in Fig. 8;
Fig. 11 is a cross-sectional view of the base of the reusable catheter product shown in Fig. 8;
Fig. 12 is a partial cross-sectional view of the reusable catheter product shown in Fig. 8.

### Description

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

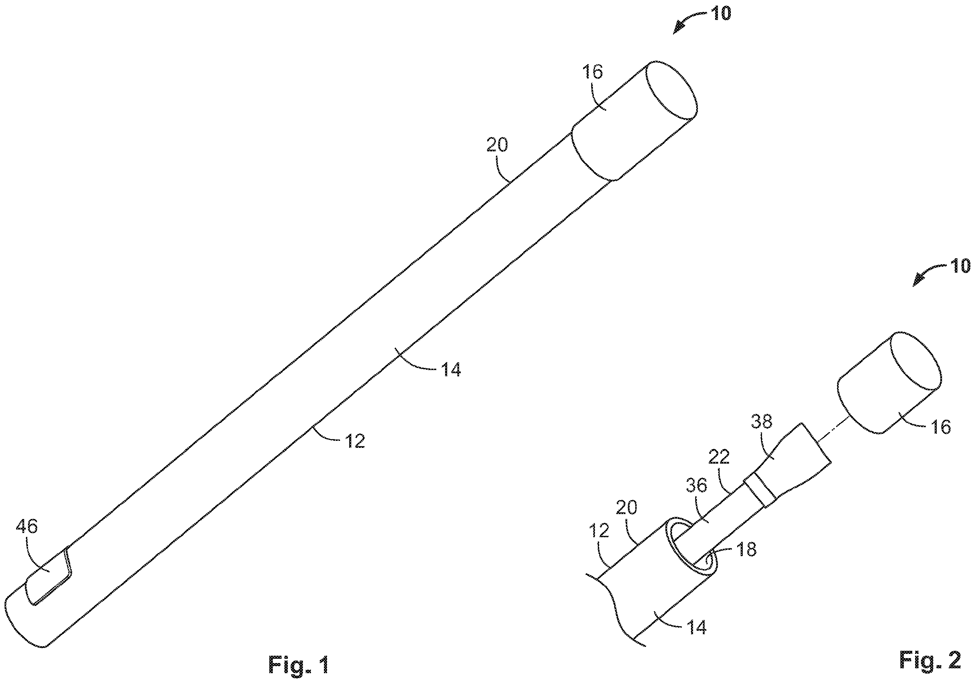

Reusable urinary catheter products according to the present disclosure and their individual components may be variously configured without departing from the scope of the present disclosure, but in one embodiment, a reusable urinary catheter product 10 is configured as shown in Fig. 1.

Figs. 1-3 show a reusable urinary catheter product 10. The product 10 includes a storage and sterilization device, such as a case 12. The case 12 includes a body 14 and re-closable cap 16. The re-closable cap 16 and body 14 may be attached to one another in any manner that allows the cap 16 to be removed and reattached to the body 14 to open and close the opening 18 (Fig. 2) in the distal end 20 of the body 14. For example, the re-closable cap 16 may be attached by threads, snap-fit, friction fit, or the like. Referring to Fig. 2, the cap 16 is removed from the body 14 to open the opening 18 of the case 12 so that the user may access the catheter 22 stored within the case 12. Optionally, the cap 16 also may be tethered to the body 14 by a tether (not shown), so that when the re-closable cap 16 is removed from the body 14 to open the case 12, the cap 16 is tethered to the body 14 to reduce the risk of dropping or losing the cap 16.

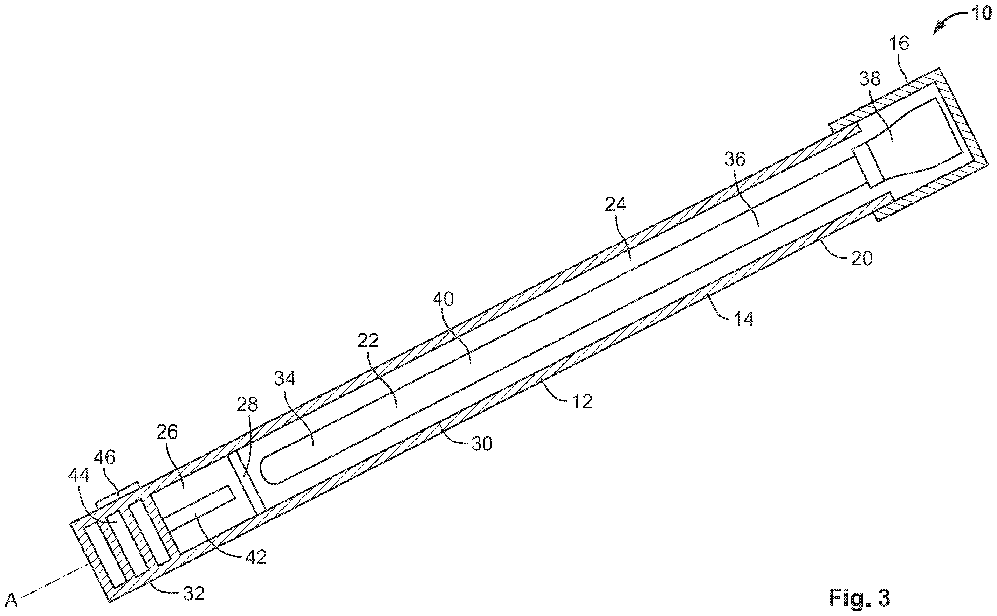

Referring to Fig. 3, the body 14 of the case 12 may be an elongated body that defines a first compartment 24 and a second compartment 26 separated by a barrier 28. In the illustrated embodiment, the body may be generally cylindrical. In other embodiments, the body may have a polygonal cross-sectional shape. The first compartment 24 is at least partially defined by a portion 30 of the body 14 that is distal of the barrier 28, and the second compartment 26 is at least partially defined by a portion 32 of the body 14 that is proximal of the barrier 28. In the illustrated embodiment, the second compartment is defined or located at the proximal end portion of the case. Also, in the illustrated embodiment, the plane of the barrier 28 extends generally perpendicular to the longitudinal axis A of the case 1 2/body 14.

The first compartment 24 is configured to hold the catheter 22, and optionally, a liquid or fluid. The catheter 22 includes a proximal insertable end 34 and a distal end 36. A drainage member 38 may be associated with the distal end 36 of the catheter 22. The catheter 22 may be a hydrophilic catheter that includes a lubricious hydrophilic coating on the catheter shaft 40. When the hydrophilic coating is hydrated, it is activated and becomes lubricious so easing insertion and removal during catheterization.

When a fluid is included in the first compartment 24, the fluid may be a sterilization fluid, a hydration fluid or both. The sterilization fluid may be any suitable sterilization fluid, and when the catheter is a hydrophilic catheter, the sterilization fluid also may serve as a hydration medium that hydrates the hydrophilic material of the coating. For example, the fluid may include one or more of chlorhexidine, FimH inhibitors, antibacterial materials, nanoparticles with bactericidal effects, oxazolidinones, antibacterial proteins, honey, glucose oxidase, silver, and a surfactant configured to loosen a biofilm from the catheter.

The second compartment 26 includes a sterilizing light source, such as a UV light source 42. The second compartment 26 may also include a power source 44. The power source 44 also could, alternatively, be located in a different location from the second compartment 26. The UV light source 42 may include one or more light emitting diodes, and the power source 44 may include batteries, which may be rechargeable batteries. Sterilizing light, such as UV light, emitted from the light source 42, is directed toward the catheter 22 and is used to sterilize or disinfect the catheter. The barrier 28 separating the first and second compartments 24 and 26 allows the passage of sterilizing light, therethrough. Thus, the light travels from the second compartment 26 through the barrier 28 and into the first compartment 24 so that the catheter 22 is exposed to the sterilizing light. The barrier 28 may be transparent or translucent. The barrier 28 may be made of glass or plastic. Optionally, the case 12 may include reflective material (not shown) that reflects the UV light within the case 12. For example, the inner wall of the body 14 and/or cap 16 may be lined with a reflective material. The reflective material may be continuous or may be segments of material that are selectively placed on the inner wall of the case 12.

As shown in Figs. 1 and 3, the case 12 may include a switch 46 to activate the light source 42 to emit sterilizing light. The switch 46 may protrude through the outer surface of the case 12 or otherwise be accessible so that it can be operated by the user. The switch 46 could be, for example, a push button or knob that is operated by the user's fingers. Alternatively, the switch 46 may be associated with the cap 16, such that when the cap 16 is reattached to the body 14, the switch activates the light source 42. The switch 46, light source 42, power source 44, and/or a timer (not shown) may be configured so that the light source 42 emits sterilizing light for a selected period of time, such as a time sufficient to disinfect the catheter 22.

In any of the embodiments disclosed herein, the catheter may include an outer surface containing titanium oxide. The titanium oxide may be integrated with the polymer forming the catheter. Alternatively, the titanium oxide may be included in a coating on the surface of the catheter 22. For example, the coating may be a hydrophilic coating containing titanium oxide. The combination of the titanium oxide with UV light sterilization can result in faster sterilization times and/or reduced bacterial attachment or colonization on the catheter.

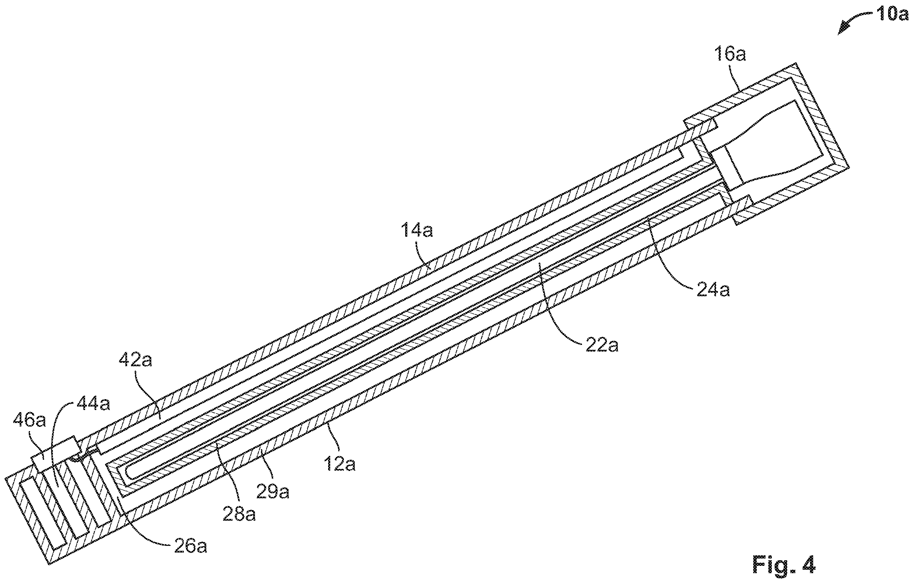

Fig. 4 illustrates an alternative embodiment of a catheter product 10a having a case 12a for storing and sterilizing a catheter 22a. Similar to that of Figs. 1-3, the case 12a includes a body 14a and a cap 16a. The body 14a includes a first compartment 24a for holding the catheter 22a, and optionally, a sterilization and/or hydration fluid. The body 14a also includes a second compartment 26a containing a sterilizing light source 42a, such as a UV light source, and power source 44a. The first and second compartments 24a and 26a are separated by a barrier 28a that allows the passage of sterilizing light therethrough. The barrier 28a may be made of the same materials as the barrier 28 described above.

The first and second compartments 24a and 26a are at least partially defined by the barrier 28a. In the illustrated embodiment, the first compartment 24a is concentric with the second compartment 26a, wherein the first compartment 24a is substantially formed by the barrier 28a. The second compartment 26a is defined between the barrier 28a and the wall 29a of the body 14a, wherein the barrier 28a forms an inner wall of the second compartment 26a and the wall 29a of the body 14a forms an outer wall of the compartment 26a.

The sterilizing light source 42a and power source 44a may be similar to that described above with respect to Figs. 1-3. In the illustrated embodiment, the light source 42a extends along the wall of the body 14a of the case 12a, and may be coextensive or at least partially coextensive with the catheter shaft. Similar to above, the case 12a may include a switch 46a to activate the UV light source. The switch 46a may be activated by the user's fingers or may be associated with the closing of the cap 16a. Additionally, the light source 42a, switch 46a, power source 44a and/or timer may be configured such that the light source 42a emits light for a selected period of time.

Referring back to Figs. 1-3, in use, the user removes cap 16 from the body 14 to open opening 18 of the case 12. As shown in Fig. 2, when the cap 16 is removed, the drainage member 38 of the catheter 22 is presented. The user removes the catheter 22 from the case 12. If a sterilization or hydration fluid is present in first compartment 24, optionally, the user removes the fluid from the compartment 24. For example, the user may pour the fluid out of the compartment 24 into a sink or toilet. The user then performs catheterization. After catheterization, optionally, the user may rinse the catheter 22, if a clean water supply is available. The user then places the catheter 22 back into the case 12, and if a sterilization fluid or hydration fluid is being employed, the user may add fluid to the case 12. The sterilizing light source 42 is activated to emit sterilizing light, such as UV light. The light source 42 may be activated by switch 46 or may be activated by attachment of cap 46. After a select period of time, the light source 42 turns off. The catheter product 10 may include a visual or audio indicator indicating to the user that the catheter 22 has been sufficiently sterilized and is ready for reuse. The indicator may be, for example, an indicator light.

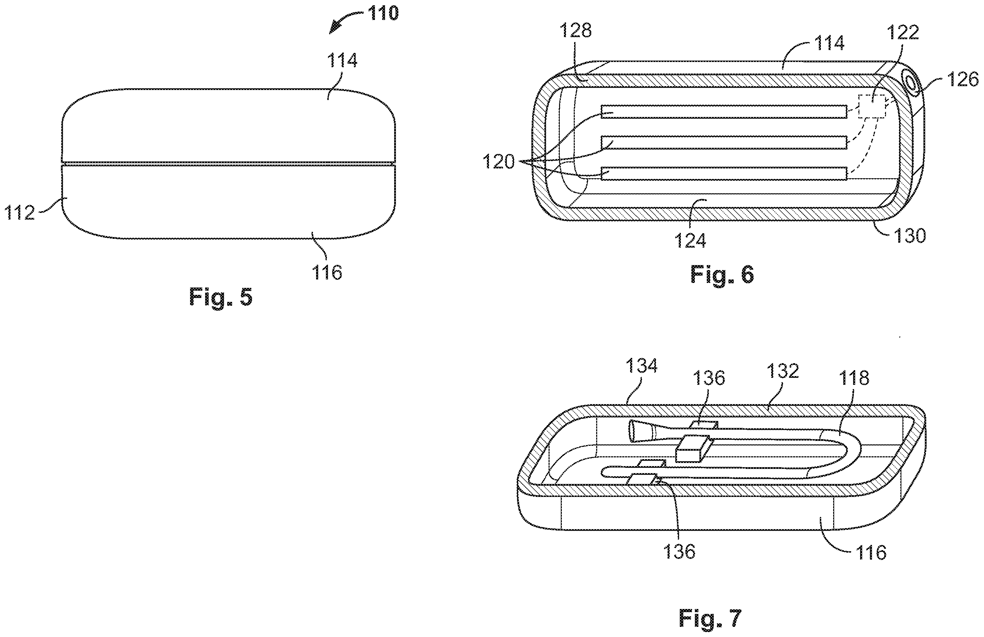

Figs. 5-7 illustrate another embodiment of a reusable catheter product 110. The reusable catheter product includes a case 112. The case 112 may include a top portion 114 and a bottom portion 116 that define a re-closable compartment that holds a catheter 118, a sterilizing light source 120 and, optionally, a sterilization and/or hydration fluid (not shown). The catheter 118 and the sterilization fluid may be any of those described herein. In the illustrated embodiment, the top and bottom portions 114 and 116 may be configured so as to be completely separated when the compartment is opened. Alternatively, the top and bottom portions 114 and 116 may have a clamshell configuration wherein the top and bottom portions are hingedly connected to each other.

Fig. 6 illustrates the top portion 114 of the case 112, and Fig. 7 illustrates the bottom portion 116 of the case 112. It should be understood that the terms "top" and "bottom" are being used for a convenient description of the case, and that the top and bottom portions could be interchanged. Referring to Fig. 6, the top portion 114 of the case 112 includes the sterilizing light source 120 and a power source 122. The sterilizing light source 120 may be associated with the bottom surface 124 of the top portion 114. The sterilizing light source 120 may be located behind a translucent or transparent barrier. The light source 120 and power source 122 may be any of the light sources and power sources disclosed herein. In the illustrated embodiment, the power source 122 may include rechargeable batteries and include a port 126 that may be connected to a power cord (not shown) for recharging the batteries. The case 112 may also include a switch (not shown) for activating the UV light source. The switch may be accessible and activated by the user. Alternatively, the switch may be configured so that the light source 120 is activated upon the closing of the case 112. Additionally, the light source 120, power source 122, switch and/or a timer may be configured so that the light source emits light for a select period of time.

In the illustrated embodiment, the case 112 is held in the closed configuration by magnetic material. As shown in Fig. 6, the top portion 114 has a magnetic segment 128 that extends about the periphery or rim 130 of the top portion 114. Referring to Fig. 7, the bottom portion 116 may have a magnetic segment 132 extending about the periphery or rim 134 of the bottom portion 116. The magnetic segment 128 of the top portion 114 and the magnetic segment 132 of the bottom portion 116 are aligned, coextensive and/or commensurate when the case 112 is in a closed configuration. In one embodiment, both the top and bottom portions 114 and 116 include magnetic segments. Alternatively, one of the top and bottom portions 114 and 116 includes a magnetic segment and the other of the portions includes a segment attracted to the magnetic material.

Referring to Fig. 7, the bottom portion 116 is configured to hold the catheter 118 in a manner that allows the catheter to be removed and reattached to the bottom portion 116. In the illustrated embodiment, the bottom portion 116 includes brackets or mounts 136 for releasably holding the catheter 118. The mounts 136 may be U-shaped clips that hold a portion of the catheter 118 by friction or snap fit. In the illustrated embodiment, the mounts 136 hold the catheter in a curved configuration. Alternatively, or in addition to the mounts 136, the bottom portion 116 also may include grooves to assist in holding the catheter.

In use, the catheter is removed from the case for catheterization, and then inserted back into the case for sterilization in a similar process as described above with respect to the embodiment of Figs. 1-3.

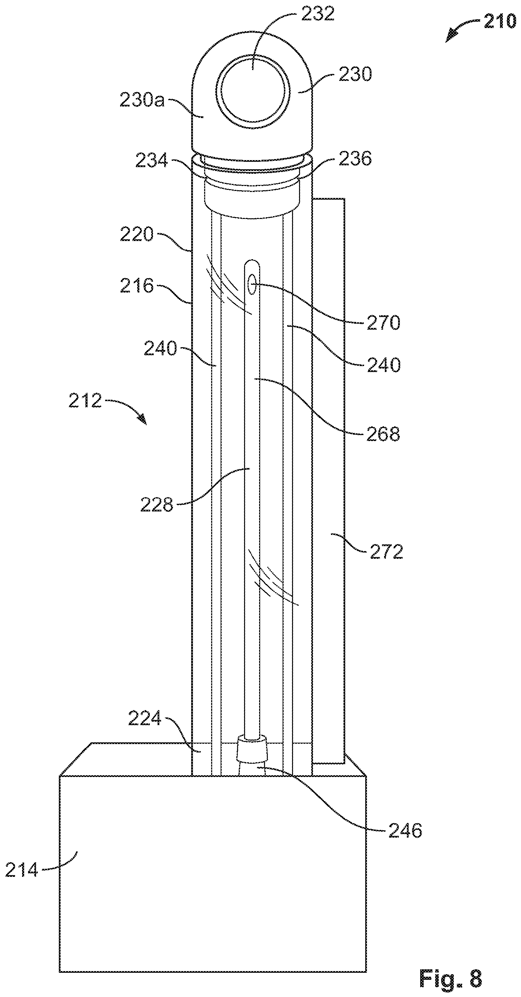

Figs. 8-12 illustrate another embodiment of a reusable catheter product 210. The reusable catheter product includes a sterilizing and storing device 212. The device 212 includes a base 214 and a sterilization chamber 216. The sterilization chamber 216 may be removably engageable with the base 214. Alternatively, the sterilization chamber 216 may be permanently attached to the base 214.

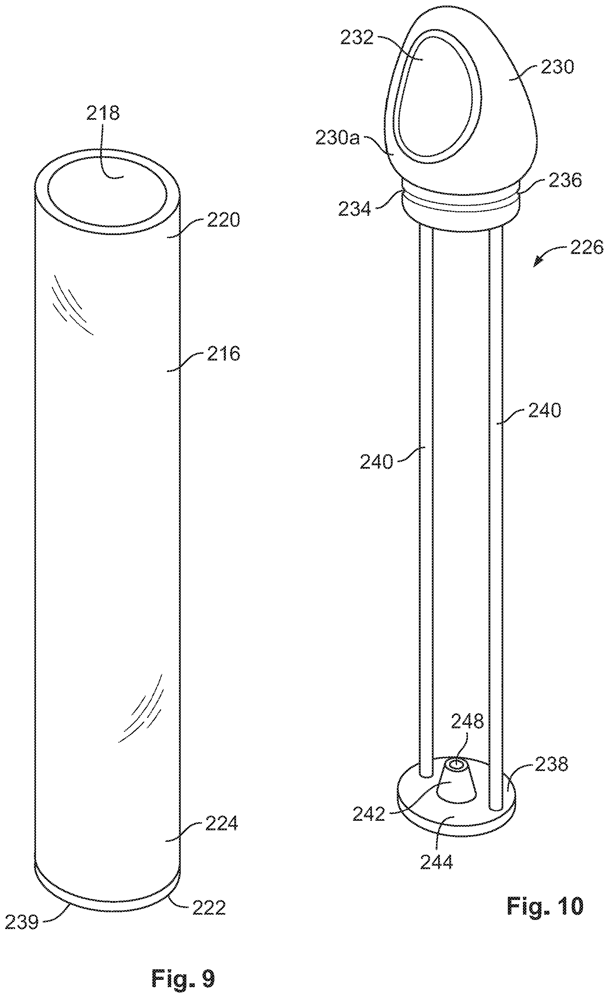

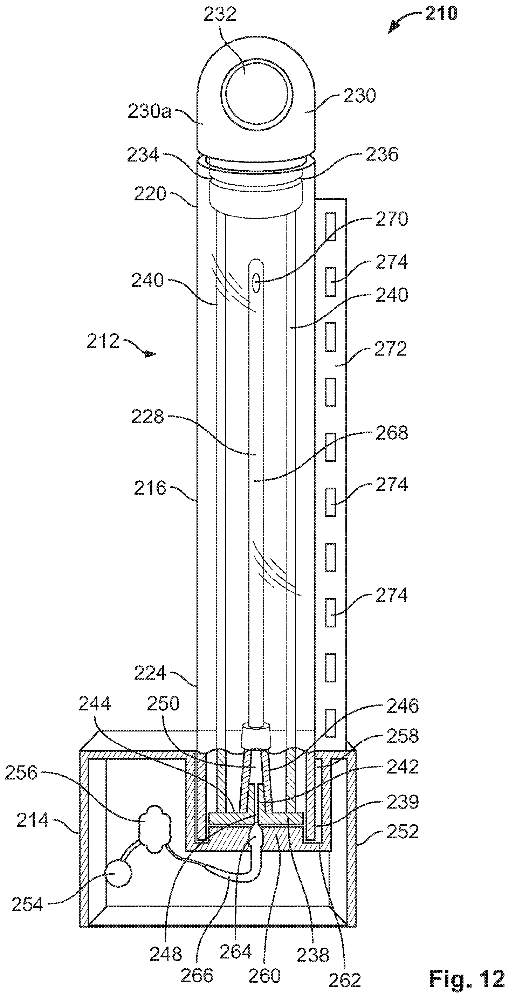

Referring to Figs. 8-10, in the illustrated embodiment, the chamber 216 has a generally cylindrical shape that defines an inner cavity. Alternatively, the chamber 216 may have other shapes. The chamber 216 also includes an opening 218 in the top end 220 and an opening 222 in the bottom end 224. A cradle 226 (Fig. 10) is inserted into and located within the chamber 216 (Figs. 8 and 12). The cradle 226 is configured to hold a catheter 228 within the chamber 216. The cradle 226 includes a top section 230 that defines a chamber cap 230a which defines the top of the cavity of the chamber 216. The chamber cap 230a includes a handle, such as the illustrated ring-shaped member 232, that may be grasped by the user to manipulate the cradle 226. The chamber cap 230a may also include a seal 234 for creating a fluid tight seal between the cap 230a and the chamber 216, when the cradle 226 is inserted into the chamber 216. The seal may be, for example, an O-ring within a groove 236.

The cradle 226 also includes a bottom section 238 that is connected to the top section 230. In the illustrated embodiment, the top section 230 and the bottom section 238 are connected by two rods 240 extending between the two sections. In other embodiments, the top and bottom sections 226 and 238 may be connected in any suitable manner.

Referring to Fig. 12, when the cradle 226 is inserted into the chamber 216, the bottom section 238 may define the bottom of cavity of the chamber 216. Additionally, the bottom section 238 may form a fluid tight seal with the chamber 216. Furthermore, the bottom section 238 may be axially recessed from the bottom edge 239 of the chamber 216. Referring to Figs. 10 and 12, the bottom section 238 of the cradle 226 includes a boss 242 extending upward from the top surface 244 of the bottom section 238. The boss 242 is configured to engage the distal end of a drainage member 246 of the catheter 228. The boss 242 defines a lumen 248 that extends through the bottom section 238 and allows the passage of flushing fluid therethrough and into the lumen 250 of the drainage member 246. In the illustrated embodiment, the boss 242 includes a cone or truncated cone shape. In other embodiments, the boss 242 may be any suitable shape.

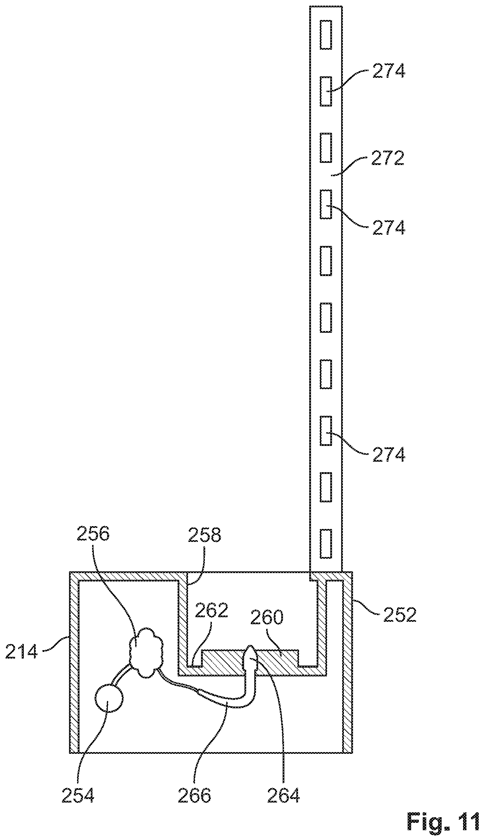

Turning to Figs. 11 and 12, the base 214 includes a housing 252 that houses a fluid supply 254 and a pump 256. The base 214/housing 252 also includes a cavity 258 that accepts the bottom 224 of the chamber 216. A mounting member 260 extends upward from the bottom surface 262 defining the cavity 258. Referring to Fig. 12, when the chamber 216 is inserted into the cavity 258, the mounting member 260 is accepted into the recess defined by the bottom section 238 of the cradle 226 and the bottom of the chamber 216. The mounting member 260 includes a lumen 264 that is aligned within and in fluid communication with the lumen 248 of the boss 242 of the bottom section 238 of the cradle 226.

Still referring to Fig. 12, a fluid flow path 266 extends between the pump 256 and lumen 264 of the mounting member 260 of the cavity 258. The pump 256 pumps flushing fluid from the fluid supply 254, through the fluid flow path 266 and lumens 264 and 248 into the lumen 250 of the drainage member 246 of the catheter 228. The fluid flow path 226 and lumens 264 and 248 serve as a fluid conduit between the fluid supply 254 and the drainage member 246 of the catheter 228. The flushing fluid flows through the catheter shaft 268 and out of the eye 270 in the proximal end of the catheter 228 to disinfect and remove microbials and particles from the inner surface of the lumen of the catheter shaft. The flushing fluid may be any of the sterilization and/or hydration fluids disclosed herein.

Optionally, the base 214 may also include a tower 272 extending upward from the base. When the chamber 216 is engaged with the base 214, the tower also may extend at least partially along the length of the chamber 216. The tower 272 includes a sterilizing light source 274. For example, the tower 272 may include one or more light emitting diodes that emit sterilizing light, such as UV light. When a sterilizing light source is included with the base 214, the chamber 216 may be made of a glass or plastic that is transparent, translucent or otherwise allows the sterilizing light to pass through the chamber 216 so that the catheter 228 is exposed to such light.

In use, the user removes the cradle 226 from the chamber 216 and removes the catheter 228 from the cradle. Catheterization is then preformed. Optionally, after catheterization the user rinses the catheter 228 with water. The catheter 228 is then placed back in the cradle 226 with the drainage member 246 engaged with the boss 242. The cradle 226 is then placed back into the chamber 216. If the chamber 216 was removed from the base 214, the chamber 216 is placed into the cavity 258 of the base 214 for receiving the chamber. The pump 256 is then activated to flow flushing fluid from the fluid supply 254 through the fluid flow passageway 266 and lumens 264 and 248 into the lumen 250 of the drainage member 246. The flushing fluid flows through the catheter 228 and out of the eyes 270 in the proximal end of the catheter. If a sterilizing light source 274 is included, the light source 274 is activated for a select amount of time to further sterilize the catheter 228 with sterilizing light. After sterilization is completed the catheter is ready for reuse.

In addition to sterilizing the catheter in the catheter products described herein, the light source may be used to refresh or replenish the hydrophilic coating of the catheter. Hydrophilic catheter coatings are formed from a hydrophilic polymer. In one embodiment the sterilization fluid or hydration medium may contain a hydrophilic polymer in the fluid/medium wherein the hydrophilic polymer is the same polymer as that in the coating or one that is compatible with the hydrophilic polymer of the coating. When the sterilization fluid or hydration medium comes into contact with the hydrophilic coating of the catheter, some of the hydrophilic polymer from the fluid/medium remains on or becomes entangled with the polymer of the hydrophilic coating. Exposure to the sterilizing light source promotes or initiates cross-linking between the hydrophilic polymer of the fluid/medium and the hydrophilic coating of the catheter, thereby refreshing or replenishing the hydrophilic coating with new or additional polymer material.

It will be understood that the embodiments described above are illustrative of some of the applications of the principles of the present subject matter.

## Claims

1. A reusable urinary catheter product (10, 10a), comprising:
a case (12, 12a) comprising a body (14, 14a) and a re-closable cap (16, 16a), the body (14, 14a) defining a first compartment (24, 24a) and a second compartment (26, 26a), the first compartment (24, 24a) and second compartment (26, 26a) being separated by a barrier (28, 28a) that allows the passage of sterilizing light;
a urinary catheter (22, 22a) located in the first compartment (24, 24a); and
a sterilizing light source (42, 42a) located in the second compartment (26), wherein sterilizing light emitted from the light source (42, 42a) passes through the barrier (28, 28a) and into the (26, 26a) second compartment.

2. The product of claim 1, wherein the body (14) has an elongated shape and has a proximal end and a distal end, the sterilizing light source (42) being located at one of the proximal and distal ends of the body (14).

3. The product of claim 2, wherein the barrier (28) includes a plane that is perpendicular to the longitudinal axis of the body (14), and the first compartment (24) is defined by a portion of the body (14) that distal of the barrier (28) and the second compartment (26) is defined by a portion of the body (14) that is proximal of the barrier (28).

4. The product of claim 1, wherein the first compartment and the second compartment (24a, 26a) are concentric.

5. The product of any one of the preceding claims, wherein the barrier (28, 28a) is transparent or translucent.

6. The product of any one of the preceding claims, wherein the (28, 28a) barrier is comprised of a plastic or glass.

7. The product of any one of the preceding claims, wherein the sterilizing light source (42, 42) comprises one or more light emitting diodes.

8. The product of any one of the preceding claims, wherein the urinary catheter (22, 22a) has a titanium oxide coating.

9. The product of claim 8, wherein the urinary catheter (22, 22a) includes a lubricious hydrophilic coating and the lubricious hydrophilic coating includes the titanium oxide.

10. The product of any one of claim claims 1-8, wherein the urinary catheter (22, 22a) includes a lubricious hydrophilic coating.

11. The product of any one of the preceding claims, further including a fluid located within the first compartment (24, 24a).

12. The product of claim 11, wherein the fluid comprising a sterilization or hydration fluid.

13. The product of claim 12, wherein the fluid comprises at least one of: an antiseptic, chlorhexidine, FimH inhibitors, antibacterial materials, nanoparticles with bactericidal effects, oxazolidinones, antibacterial proteins, honey, glucose oxidase, silver, and a surfactant configured to loosen a biofilm from the catheter.

14. The product of any one of the preceding claims, further including a power source (44, 44a) for powering the sterilizing light source (42, 42a).

15. The product of any one of the preceding claims, wherein the case (12, 12a) further includes reflective material that reflects sterilizing light.

## Patentansprüche

1. Wiederverwendbares Harnkatheterprodukt (10, 10a), umfassend:
ein Gehäuse (12, 12a), das Folgendes umfasst: einen Körper (14, 14a) und eine wiederverschließbare Kappe (16, 16a), wobei der Körper (14, 14a) ein erstes Fach (24, 24a) und ein zweites Fach (26, 26a) definiert, wobei das erste Fach (24, 24a) und das zweite Fach (26, 26a) durch eine Barriere (28, 28a) getrennt sind, die den Durchgang von Sterilisierungslicht ermöglicht;
einen Harnkatheter (22, 22a), der sich in dem ersten Fach (24, 24a) befindet; und eine Sterilisierungslichtquelle (42, 42a), die sich in dem zweiten Fach (26) befindet, wobei das von der Lichtquelle (42, 42a) emittierte Sterilisierungslicht durch die Barriere (28, 28a) und in das zweite Fach (26, 26a) hindurchgeht.

2. Produkt nach Anspruch 1, wobei der Körper (14) eine längliche Form aufweist und ein benachbartes Ende und ein distales Ende aufweist, wobei sich die Sterilisierungslichtquelle (42) an einem der proximalen und distalen Enden des Körpers (14) befindet.

3. Produkt nach Anspruch 2, wobei die Barriere (28) eine Ebene beinhaltet, die senkrecht zu der Längsachse des Körpers (14) verläuft, und das erste Fach (24) durch einen Abschnitt des Körpers (14) definiert ist, der distal zu der Barriere (28) liegt, und das zweite Fach (26) durch einen Abschnitt des Körpers (14) definiert ist, der benachbart zu der Barriere (28) liegt.

4. Produkt nach Anspruch 1, wobei das erste Fach und das zweite Fach (24a, 26a) konzentrisch sind.

5. Produkt nach einem der vorhergehenden Ansprüche, wobei die Barriere (28, 28a) transparent oder lichtdurchlässig ist.

6. Produkt nach einem der vorhergehenden Ansprüche, wobei die Barriere (28, 28a) aus Kunststoff oder Glas besteht.

7. Produkt nach einem der vorhergehenden Ansprüche, wobei die Sterilisierungslichtquelle (42, 42) eine oder mehrere lichtemittierende Dioden umfasst.

8. Produkt nach einem der vorhergehenden Ansprüche, wobei der Harnkatheter (22, 22a) eine Titanoxidbeschichtung aufweist.

9. Produkt nach Anspruch 8, wobei der Harnkatheter (22, 22a) eine gleitfähige hydrophile Beschichtung beinhaltet und die gleitfähige hydrophile Beschichtung das Titanoxid beinhaltet.

10. Produkt nach einem der Ansprüche 1-8, wobei der Harnkatheter (22, 22a) eine gleitfähige hydrophile Beschichtung beinhaltet.

11. Produkt nach einem der vorhergehenden Ansprüche, das ferner ein Fluid beinhaltet, das sich innerhalb des ersten Fachs (24, 24a) befindet.

12. Produkt nach Anspruch 11, wobei das Fluid ein Sterilisierungs- oder Hydratisierungsfluid umfasst.

13. Produkt nach Anspruch 12, wobei das Fluid mindestens eines von Folgendem umfasst: ein Antiseptikum, Chlorhexidin, FimH-Inhibitoren, antibakterielle Materialien, Nanopartikel mit bakterizider Wirkung, Oxazolidinone, antibakterielle Proteine, Honig, Glucoseoxidase, Silber und ein Tensid, das dazu konfiguriert ist, einen Biofilm vom Katheter zu lösen.

14. Produkt nach einem der vorhergehenden Ansprüche, das ferner eine Stromquelle (44, 44a) zum Versorgen der Sterilisierungslichtquelle (42, 42a) beinhaltet.

15. Produkt nach einem der vorhergehenden Ansprüche, wobei das Gehäuse (12, 12a) ferner ein reflektierendes Material beinhaltet, das Sterilisierungslicht reflektiert.

## Revendications

1. Produit de cathéter urinaire réutilisable (10, 10a), comprenant :
un boîtier (12, 12a) comprenant un corps (14, 14a) et un capuchon refermable (16, 16a), le corps (14, 14a) définissant un premier compartiment (24, 24a) et un second compartiment (26, 26a), le premier compartiment (24, 24a) et le second compartiment (26, 26a) étant séparés par une barrière (28, 28a) qui permet le passage d'une lumière stérilisante ;
un cathéter urinaire (22, 22a) situé dans le premier compartiment (24, 24a) ; et
une source de lumière stérilisante (42, 42a) située dans le second compartiment (26), dans lequel la lumière stérilisante émise par la source de lumière (42, 42a) traverse la barrière (28, 28a) et pénètre dans le second compartiment (26, 26a).

2. Produit selon la revendication 1, dans lequel le corps (14) a une forme allongée et présente une extrémité proximale et une extrémité distale, la source de lumière stérilisante (42) étant située à l'une des extrémités proximale et distale du corps (14).

3. Produit selon la revendication 2, dans lequel la barrière (28) comprend un plan qui est perpendiculaire à l'axe longitudinal du corps (14), et le premier compartiment (24) est défini par une partie du corps (14) qui est distale de la barrière (28) et le second compartiment (26) est défini par une partie du corps (14) qui est proximale de la barrière (28).

4. Produit selon la revendication 1, dans lequel le premier compartiment et le second compartiment (24a, 26a) sont concentriques.

5. Produit selon l'une quelconque des revendications précédentes, dans lequel la barrière (28, 28a) est transparente ou translucide.

6. Produit selon l'une quelconque des revendications précédentes, dans lequel la barrière (28, 28a) est constituée d'un plastique ou d'un verre.

7. Produit selon l'une quelconque des revendications précédentes, dans lequel la source de lumière stérilisante (42, 42) comprend une ou plusieurs diodes électroluminescentes.

8. Produit selon l'une quelconque des revendications précédentes, dans lequel le cathéter urinaire (22, 22a) a un revêtement en oxyde de titane.

9. Produit selon la revendication 8, dans lequel le cathéter urinaire (22, 22a) comprend un revêtement hydrophile lubrifiant et le revêtement hydrophile lubrifiant comprend l'oxyde de titane.

10. Produit selon l'une quelconque des revendications 1 à 8, dans lequel le cathéter urinaire (22, 22a) comprend un revêtement hydrophile lubrifiant.

11. Produit selon l'une quelconque des revendications précédentes, comprenant également un fluide situé à l'intérieur du premier compartiment (24, 24a).

12. Produit selon la revendication 11, dans lequel le fluide comprend un fluide de stérilisation ou d'hydratation.

13. Produit selon la revendication 12, dans lequel le fluide comprend au moins l'un des éléments suivants : un antiseptique, de la chlorhexidine, des inhibiteurs de FimH, des matériaux antibactériens, des nanoparticules à effets bactéricides, des oxazolidinones, des protéines antibactériennes, du miel, de la glucose oxydase, de l'argent et un tensioactif conçu pour détacher un biofilm du cathéter.

14. Produit selon l'une quelconque des revendications précédentes, comprenant également une source d'alimentation (44, 44a) pour alimenter la source de lumière stérilisante (42, 42a).

15. Produit selon l'une quelconque des revendications précédentes, dans lequel le boîtier (12, 12a) comprend également un matériau réfléchissant qui réfléchit la lumière stérilisante.
